# EUROPEAN PATENT SPECIFICATION

(11) **EP 1 170 591 B1**
(45) Date of publication and mention of the grant of the patent: **22.06.2005**
(21) Application number: 01115703.9
(22) Date of filing: 05.07.2001
(51) Int. Cl.: G01N 33/543, G01N 33/50

(54) **Methods for identifying combinations of entities as therapeutics**
Verfahren zur Identifizierung von Verbindungen der Wesens als Therapeutika
Méthodes pour l'identification des combinaisons d'entités comme thérapeutiques

(30) Priority: 07.07.2000 US 611835
(43) Date of publication of application: 09.01.2002
(73) Proprietor: CombinatoRx, Incorporated, Boston MA 02118 (US)
(72) Inventor: Stockwell, Brent R., Boston, MA 02114 (US); Borisy, Alexis, Boston, MA 02114 (US); Foley, Michael A., Chestnut Hill, MA 02467 (US)
(74) Representative: Bösl, Raphael, Dr. rer. nat., Dipl.-Chem.

(56) References cited:
- WO-A-98/57174
- US-A- 5 374 536
- US-A- 5 989 835
- STOCKWELL B R ET AL: "HIGH-THROUGHPUT SCREENING OF SMALL MOLECULES IN MINIATURIZED MAMMALIAN CELL-BASED ASSAYS INVOLVING POST-TRANSLATIONAL MODIFICATIONS" CHEMISTRY AND BIOLOGY, CURRENT BIOLOGY, LONDON, GB, vol. 6, no. 2, February 1999 (1999-02), pages 71-83, XP000865522 ISSN: 1074-5521

## Description

### Background of the Invention

Many disease states are associated with a multitude of phenotypic changes. This has long been apparent in the clinic, but recent advances in genomics have confirmed this observation at the molecular level. Expression profiling of cancer cells, for example, has revealed hundreds of changes in gene expression caused by multiple somatic mutations. Furthermore, human cells and tissues have evolved homeostatic mechanisms such that they often contain redundant and self-buffering signaling systems. Natural signals causing a change in cellular state are often sent not to a single target, but to the correct combination of targets. Thus, modest changes in multiple variables can have a highly specific effect.

In contrast, for historical and technological reasons, the pharmaceutical, chemical, and biological communities have focused on single, individual molecules that cause biologic effects. This historical paradigm has resulted in the identification of small organic molecules that affect specific proteins, providing valuable reagents for both biology and medicine. These molecules are also useful as probes of the biological function of proteins that may have therapeutic relevance and have been effective at elucidating signal transduction pathways by acting as chemical protein knockouts, thereby causing a loss of protein function. Additionally, due to the interaction of these small molecules with particular biological targets and their ability to affect specific biological functions, they may also serve as candidates for the development of therapeutics.

Because it is impossible to predict which small molecules will interact with a biological target, intense efforts have been directed toward the generation of large numbers of small organic molecules. These are then gathered into what are called "libraries" of such compounds, with the goal of building a "diverse library" with the appropriate desired characteristics. Such a diverse library may be built from a preexisting collection of small molecules or may be generated using "combinatorial chemistry." These libraries can be linked to sensitive screens to identify active molecules (Stockwell et al. Chem. Biol. 1999, 6, 71-83).

In many cases, researchers have developed biased libraries, in which all members share a particular characteristic, such as an ability to interact with a target protein, or a characteristic structural feature designed to mimic a particular aspect of a class of natural compounds. For example, a number of libraries have been designed to mimic one or more features of natural peptides. Such "peptidomimetic" libraries include phthalimido libraries (WO 97/22594), thiophene libraries (WO 97/40034), and benzodiazepine libraries (US Patent No. 5,288,514). One library that has structural features reminiscent of natural products and that is compatible with miniaturized cell-based assays has been synthesized (Tan et al. J. Am. Chem. Soc. 1998, 120, 8565).

The modem drug discovery process is largely built upon an ability to assay rapidly compounds for their effects on biological processes. In the pharmaceutical industry, attention has been focused on identifying compounds that block, reduce, or even enhance the interactions between biological molecules. Specifically, in biological systems, the interaction between a receptor and its protein ligand often may result, either directly or through some downstream event, in either a deleterious or beneficial effect on that system, and consequently, on a patient for whom treatment is sought. Accordingly, researchers have long sought compounds that reduce, block, or even enhance such receptor/ligand interactions.

High throughput screening systems were designed to overcome the practical limitations on throughput for existing biochemical and cell-based assays. Traditional syntheses of organic compounds and traditional biological assays require significant time, labor, and skill. Screening the maximum number of compounds necessitates reducing the time and labor requirements associated with each screen.

High throughput screening of diverse collections of molecules has thus played a central role in the search for lead compounds for the development of new pharmacological agents. The inputs to these high throughput screens are libraries of compounds that have been assembled from preexisting chemically synthesized molecules (such as from a pharmaceutical company's proprietary library), natural products (such as microbial fermentation broths), and from novel libraries generated by combinatorial chemistry techniques (Tan et al. J. Am. Chem. Soc. 1998, 120, 8565). The libraries consist of up to a million compounds, which increases the likelihood of finding one compound with desirable properties to serve as a lead drug candidate (Tan et al J. Am. Chem. Soc. 1999, 121, 9073-9087).

Enhancing the traditional paradigm of drug discovery, combinatorial chemistry has resulted in a dramatic increase in the number of compounds that are available for screening, and human genome research has uncovered large numbers of new molecular targets for screening. Screens may use these new targets in a variety of ways, searching for enzyme inhibitors, receptor agonists or antagonists. The traditional goal is to find compounds that reduce, block, or enhance a single crucial interaction in a biological system (Weber et al., Angew. Chem. Int. Ed. Engl., 1995, 34, 2280-2282). Additionally, a number of researchers are adapting phenotype-based assay systems, where the screening is performed on whole, living cells, and the readout of the screen is some detectable property of the cell (Stockwell et al. Chem. Biol. 1999, 6, 71-83; Mayer et al. Science, 1999, 286, 971-974).

The high throughput screening methods developed to date have been designed based on the "one-drug-one-target" paradigm that dominates the pharmaceutical industry. For historical reasons, and because of regulatory considerations and perceived risk factors, the modern drug discovery process primarily looks to find one active molecule at a time to serve as a drug candidate. Clinicians have long recognized that the "one-drug-one-target" approach is not sufficient for the treatment of many diseases. They have tested some obvious combinations of agents that treat the same condition, or that have a clear logical connection. In HIV therapy and chemotherapy, for example, combinations of multiple active agents have become *de rigeur*. One of the most promising drugs of all times is a combination--Premarin, which is used to treat the complex changes in females after menopause, is composed of over 22 separate and important components.

As another example, WO 98/57174 discloses synergistic combinations of inhibitory peptides with antimicrobial compounds.

### Summary of the Invention

We have invented a powerful new method for perturbing biological systems. This entails systematically performing high throughput screens of combinations of compounds, i.e. mixtures or non-chemically bonded combinations, to discover combinations that interact synergistically in biological systems. The methods of the invention can identify effective therapeutic combinations of compounds that exhibit previously unknown therapeutic effects even where each compound in the combination may have previously have had no recognized biological effect, and even where the compounds and the combination would not, *a priori*, have been obvious candidates to use in combination.

Accordingly, the invention provides a method for screening combinations of compounds for identifying combinations of compounds that cause an effect that is different from the effect of a compound of the combination by itself, said method comprising the steps of: (a) providing whole cells of a whole cell assay, (b) contacting said whole cells with at least 100 compounds under conditions that ensure that each compound/whole cell contacting is segregated from the others, and (c) detecting or measuring effects displayed by the compounds on the whole cells, wherein said method comprises the additional following steps of: (d) selecting compounds that cause a change in said effect displayed on the whole cells relative to said effect of said whole cells not contacted with said compounds, (e) creating an array of at least 49 unique two-entity or higher order combinations from the identified compounds, (f) contacting said array of combinations of compounds with whole cells of the whole cell assay under conditions that ensure that each compound combination/whole cell contacting is segregated from the others, (g) detecting or measuring an effect displayed by the combinations of compounds on the whole cells of step (f), and (h) identifying combinations of compounds that cause an effect of step (g) that is different from the effect of a compound of the combination by itself.

In a second, related aspect, the method includes the step of creating an array of at least 200 unique combinations of two or more entities. The components of this assay can be varied as described above in connection with the first assay. In preferred embodiments, this method further includes the step of (i) repeating steps (a) through (h) at least twice, wherein, in step (a), the array of at least 200 combinations is different in each repetition. In other preferred embodiments, the array includes at least 400 or 1540 unique combinations. Where two repetitions of step (i) are carried out, preferably those occur within ten days of each other.

In a third, related aspect, the method includes the steps of (a) to (h) and (i) repeating steps (a) through (h) at least 25 times over a one week period, using a different array in each repetition. The components of this assay can be varied as described above for the previously described assays.

In a fourth, related aspect, the method includes the steps of (a) to (h) and (i) repeating steps (a) through (h) at least 100 times over a one month period, using a different array in each repetition. The components of this assay can be varied as in the previously described assays.

In a fifth aspect, the method includes the steps of (a) to (h) with creating an arry of at least 10,000 unique two-entity of higher order combinations from the identified compounds, and (i) repeating steps (a) through (h) at least twice over a period of ten days or less, wherein, in step (a), the array of at least 10,000 two-entity combinations is different in two or more repetitions.

In a sixth aspect, the effect of step (c) is the preferably the same as of the whole cells of step (g), but it may be desirable to use a different effect in each step.

For all of the assays, a number of activity read-out techniques can be used, including a cytoblot assay, a reporter gene assay, fluorescence resonance energy transfer (FRET) assays, a fluorescent calcium binding indicator dye, fluorescence microscopy, or expression profiling. The assays preferably are automated, using robotics systems and 384 and 1536 well plates. The assays may also be constructed so that parts or all of the process may be performed using microfluidics. Alternatively, the assays may be executed by using trained scientific labor and an efficient line process.

The compounds to be tested in combination according to the invention are e.g., non-polymeric organic compounds, in particular, small molecules; lipids; carbohydrates; peptides; inorganic compounds; and oligonucleotides, including DNA and RNA molecules. The compounds are preferably used in purified form, but can also be provided as components of mixtures, e.g., as natural product extracts. For example, a subset (one or more compounds) can be in purified form, each of the compounds can be employed in purified form. Preferably, each compound/whole cell contacting is in a volume of less than 200 µL, more preferably less than 100 µL, and most preferably less than 50 µL or even 25 µL. In certain circumstances, volumes as little as 10 µL or even 500 nL or less may be used. Additionally, the compounds are preferably in volumes less than 100 µL, more preferably less than 1 µL, and most preferably less than 100 nL or even 50 nL. One in the art will recognize that smaller volumes (e.g., 10 nL or even 1 nL) may be used.

Preferred whole cells are e.g., human cells, transformed cells or non-transformed cells, such as neurons, fibroblasts, smooth muscle cells, cardiac cells, skeletal muscle cells, glial cells, embryonic stem cells, hematopoeitic stem cells, mast cells, adipocytes, protozoans, bacterial cells, yeast cells, neural stem cells, and immune cells including T cells and B cells, clusters of cells, tissues, animals, and reconstituted cell-free media.

The effects displayed by desired combinations on the whole cell are preferably synergistic effects on a property of the whole cell, such as an increase or decrease in DNA synthesis. Alternatively, the combination may be additive in nature but have fewer side effects, or one entity may exert a negative, and useful effect, on another, e.g., one compound counteracting toxicity of another compound.

The compounds can be contacted with the whole cell in any sequence, i.e., one compound can be added to the whole cell, followed by the addition of a second compound, or alternatively, the two compounds can be combined prior to their being contacted with the whole cell.

The high throughput screening methods of the invention represent rapid and powerful alternatives to traditional drug discovery methods employed by the pharmaceutical industry. The invention can identify previously unknown, and therapeutically potent combinations of, e.g., small molecules, some of which may be newly synthesized and some of which may be known FDA-approved drugs. Where the effective combinations are entirely made up of two, three, four, or more drugs, all of which are already FDA approved, the new combination has the further advantage of being easily moved through the FDA approval process.

Other features and advantages of the invention will be apparent from the following detailed description, and from the claims.

### Definitions

"Combinatorial chemistry library": As used herein, a "combinatorial chemistry library" is a plurality of complex molecules, preferably reminiscent of natural products synthesized from diversifiable scaffold structures by employing different reactants at each stage of diversification of the scaffold structure.

"Diverse library": As used herein, a "diverse library" is a plurality of complex molecules that have been assembled from any of multiple potential sources, including preexisting chemically synthesized molecules (such as from a pharmaceutical company's proprietary library), natural products (such as microbial fermentation broths), and novel libraries generated by combinatorial chemistry techniques.

"Diversifiable scaffold structure": As used herein, a "diversifiable scaffold structure" is a compound synthesized from a template structure, which contains latent or active functionalities capable of being further reacted with synthetic reagents to generate at least one new functionality. As used herein a "latent functionality" is one that is present, but is temporarily inactive. Upon release with an activator reagent, the latent functionality becomes active, and is thus available for further diversification

"Test element": As used herein, a "test element" is a whole cell to which the combination of compounds is contacted, and which is then observed for the effects of the compounds. A whole cell preferably includes two or more biological moieties inside of the cell.

"Compound printing": As used herein, "compound printing" refers to the application of compounds to a surface (e.g., glass) using a high-precision robot such as that used in cDNA microarraying (J. Am. Chem. Soc. 1999, 121, 7967-7968). The compound spots can be 250 microns in diameter or smaller, and the compounds may be either covalently linked or adhering to the surface through electrostatic or hydrophobic interactions.

"Microfluidics": As used herein, "microfluidics" devices are channeled structures made by any of the methods of photolithography, including conventional photolithography (e.g. Caliper Technologies, Mountain View, CA; http://www.calipertech.com) or unconventional methods (such as soft lithography, described, e.g., in Angew. Chem. Int. Ed. Engl. 1998, 37, 550-575).

"Ink-jet": As used herein, "ink-jet" technology refers to both thermal ink-jet as well as piezoelectric spray technologies for delivery small volumes of liquids.

"Small molecule": As used herein, "small molecule" refers to an organic compound either synthesized in the laboratory or found in nature. Typically, a small molecule is characterized in that it contains several carbon-carbon bonds, and has a molecular weight of less than 1500 g/mole, although this characterization is not meant to be limiting for the purposes of the present invention. Examples of "small molecules" that occur in nature include, but are not limited to, taxol, dynemicin, and rapamycin.

### Brief Description of the Drawings

Fig. 1 is a conceptual diagram demonstrating how two different reagents could act synergistically inside of a cell, where the reagents bind to different targets within the same cell.
Fig. 2 is a conceptual diagram demonstrating how two different reagents could act synergistically inside of an organism, where the reagents bind to targets in different cells or tissues.
Fig. 3 is an example of the experimental data one would obtain in a combinatorial screen of the sort described herein. The results from five different 384 well plates are shown. The results are shown in plate format, where there are 16 rows labeled A through P, and 24 columns, labeled 1 through 24. The level of activity is shown in each well as a number, where 1 means basal activity (no effect) and 5 means an active combination is found.
Fig. 4 is a diagram of a method for performing combinatorial screening using currently commercially available technology.

### Detailed Description

The present invention provides methods of combining libraries of individual molecules in 2-, 3-, 4-, and higher order combinations in a high throughput screening system using relevant biological assays to identify effects of the molecules that are only present in their unique combinations. Such combinations may then be used to probe and study additional biological systems, may have a direct biological use, and may serve as the active molecules for novel human therapeutics or other uses in humans. These combinations may also be useful for promoting the growth, fertility, maturation, or other characteristic in specific agricultural products, including animals or plants. They may be useful in creating cosmetic products, fragrances, food preservatives, or nutritional supplements. Thus, the invention provides powerful methods for systematically performing high throughput screens of combinations of compounds to discover combinations having improved properties in biological systems.

We posit that pharmaceuticals interacting with complex systems such as human cells and tissues are likely to be most effective when they contain multiple active agents that interact with multiple molecular targets. This understanding of how pharmaceuticals operate forms one of the bases for our new strategy for how they should be designed and developed. This new approach promises to yield dramatic improvements in many existing treatments, and provide effective therapies for currently intractable diseases, particularly diseases requiring modest changes in multiple variables.

The current dominant paradigm of the pharmaceutical industry is one in which one drug is designed against one target. Our approach uses our understanding of multivariable design requirements as the centerpiece of a framework for the systematic development of a new generation of pharmaceuticals.

### Compounds to be Tested in Combination

As is mentioned above, a wide variety of compounds can be tested in combination according to the invention. These are now discussed in greater detail.

The prevalent class of compounds that are screened according to the invention are small organic molecules. The compounds can be synthetic or naturally occurring (e.g., prostaglandins, lectins, naturally occurring secondary metabolites, hormones, etc.). Large libraries of such molecules, in purified form, are available in pharmaceutical companies, chemicals companies, and academic laboratories; such libraries also can be synthesized using known combinatorial chemistry techniques. The compounds may or may not have known biological activity. The compounds and combinatorial libraries of the.invention can be synthesized from diversifiable solid support bound scaffolds, e.g., compounds and libraries that are generated from a diversifiable scaffold synthesized from a shikimic acid-based epoxyol template or from the pyridine-based template isonicotinamide. In addition to small organic molecules, other molecules that can be screened in combination according to the invention include biopolymers, including oligonucleotides (DNA and RNA); polypeptides (antibodies, enzymes, receptors, ligands, structural proteins, mutant analogs of human proteins, and peptide hormones); lipids; carbohydrates; and polysaccharides. Inorganic molecules can be screened as well, as can potentially biologically active ions such as metal ions, e.g., copper, iron, silver, zinc, magnesium, manganese, calcium, and gold ions.

### Test Elements

The biological assays used to detect the effects of the combinations will in most cases be composed of multiple components. In the assays, the test element is a whole cell, particularly where the assay is a phenotype-based assay. Such a whole cell assay provides the complete set of complex molecular interactions that are likely to form the basis of a multivariable therapeutic compound's efficacy. Other assays employ higher order biological systems such as clusters of whole cells, tissues, and animal models for multivariable combinatorial screening.

Any biological assay that is useful for assay of individual compounds is readily adapted to the combinatorial screening of the present invention. Assay measurements can include, for example, transport of a compound across the cell membrane, electrical potential, action potential generation, cell proliferation, cell death, cell specification, cell differentiation, cell migration, gene expression or protein levels (measured, e.g., by detecting mRNA, protein, or a reporter gene), enzymatic activity, phosphorylation, methylation, acetylation, translocation of a protein to the cell nucleus (or other change in protein localization), ability to resist a pathogen (e.g., a virus or a bacterium), and ability to produce an immune response. In whole organism assays, animal behavior can serve as a reporter.

In one example, the assay is a non-destructive assay (e.g., a cell-based assay in which a measurement of the effect of a compound can be obtained without harming the cells). Such an assay allows assays to be performed on multiple concentrations of multiple combinations per well. For example, compound A is added at increasing concentrations to a well and a measurement is taken after each addition of compound. When a desired concentration of compound A is reached (determined based on a desired assay response or on known properties (e.g., toxicity, solubility) of the compound), compound B is added in increasing concentrations, with an assay measurement taken after each addition. This process can be iterated many times in a single well, allowing hundreds, thousands, or even millions of assays to be performed in a single plate.

It may be desirable to perform a comparison assay to identify possible side effects of combinations. For example, combinations of compounds that are screened for their ability to kill or decrease proliferation of tumor cells can be simultaneously screened for their effect on normal, non-tumor cells. Combinations of compounds that display the desired activity on tumor cells and that have little or no effect on normal cells are preferred combinations. Combinations that appear to have an undesired effect on normal cells are less preferred. These latter combinations (or combinations of compounds having similar structures) may, however, be effective at different concentrations and should not necessarily be excluded from additional study.

### Cytoblot Assay

One method of detecting activity is the cytoblot assay. In this method, cells are seeded into wells of an assay plate. The cells are preferably adherent cells so they attach and grow on the bottom of the well. The compounds are added using the methods described above. For example, the cytoblot can be performed to detect proliferation by measuring the incorporation of BrdU. In this example, the cells are incubated for a set period of time, (e.g., 4 to 72 hours). The medium is then aspirated using, for example, a robotic liquid transfer device or a sixteen or eight channel wand. The cells are fixed by the addition of 70% ethanol and phosphate-buffered saline (PBS) at 4°C for 1 hour. The fixitive is removed and the cells are washed once with PBS. After the PBS wash, 2N HCl with 0.5% Tween 20 is added to each well for 20 minutes. The HCl is neutralized with a solution of Hank's balanced salt solution (HBSS) containing 10% by volume of 2N NaOH. This solution is removed, the cells are washed twice with HBSS and then once with PBS containing 0.5% bovine serum albumin (BSA) and 0.1% Tween 20. The wash solution is removed and anti-BrdU antibody is applied as 0.5 µg/mL mouse anti-BrdU antibody in PBS containing 0.5% bovine serum albumin (BSA) and 0.1% Tween 20. This antibody solution also contains a secondary antibody (at a dilution of 1:2000) that recognizes mouse Ig antibody (e.g., the mouse anti-BrdU antibody); this secondary antibody is conjugated to the enzyme horseradish peroxidase (HRP). After one hour of incubation, the antibody solution is removed and the cells are washed twice with PBS. After the second PBS wash, the HRP substrate (which contains luminol, hydrogen peroxide, and an enhancer such as para-iodophenol is added to each well. The amount of light in each well is then detected using either exposure to film (by placing a piece of film on top of the plate) or by reading the amount of luminescence from each well using a luminometer or luminescence plate reader using standard conditions (e.g., 0.3 seconds of exposure per well). The amount of light output from each well indicates the amount of DNA synthesis that occurred in that well. A desirable combination of agents is one in which there is either increased or decreased light output compared to a control. For example, a combination that decreases light output would be decreasing the rate of DNA synthesis and, thus, may be effective in prohibiting or preventing the proliferation of tumor cells. Alternatively, an increase in light output represents an increase in DNA synthesis. For example, one could use primary cells in, for example, a 200 X 1 combinatorial array (in which the one fixed component blocked DNA synthesis and, thus, had a toxic effect on the cells). Using this array, one could screen for a second reagent that prevented this toxic effect of the first compound. In a variety of immune deficiency diseases, the immune cells do not proliferate in response to either an allo- or an auto-antigen. Using cultured immune cells and the foregoing method, one can screen for combinations of reagents that promote the proliferation of immune cells. Alternatively, one can screen for autoimmune suppressive agents. In this example, a population of one type of immune cell (a B-cell or a T-cell that has been isolated from peripheral blood cells) is stimulated by either allo- or auto-antigen. Combinations of compounds that specifically inhibit proliferation in response to auto-antigens but not allo-antigens are useful autoimmune therapeutic candidates.

### Other Methods of Detecting Activity

The foregoing cytoblot assay is readily adapted to the detection of antigens other than BrdU. Moreover, one can detect a variety of post-translational modifications within cells. For example, an antibody against the phosphorylated version of nucleolin or histone H3 is useful for detecting cells that are in M (mitosis) phase of the cell cycle. Combinations of compounds that cause an increase in phosphorylated nucleolin or histone H3 in the cytoblot assay would therefore be combinations that arrest cells in M phase and are possible anticancer agents. One could also use a cytoblot assay to detect increases in the acetylation of, for example, histone H4 using an antibody that specifically recognizes acetylated histone H4. Compounds or combinations of compounds that cause an increase in hyperacetylation of histone H4 could also be anticancer agents.

In the foregoing examples, the procedure may be altered in that the medium is removed and a fixative (70% ethanol or 4% formaldehyde in PBS or Tris-buffered saline) is added. The membrane of the cells is then permeabilized by removing the fixative and adding a permeabilization agent (e.g., Tween 20, triton X-100, or methanol). The membrane permeabilization agent is removed, the cells are then washed with PBS or Tris-buffered saline, and then the primary antibody is added. There is usually no acid denaturation step using these other cytoblot embodiments.

### Reporter Gene Assays

Measurement of a property of the test element may be performed with the use of a reporter gene. This method provides the advantage that, once the reagents (e.g., a stably transfected cell line) are prepared, the assay is easy to perform and requires less time than, for example, the cytoblot assay. Reporter genes include a reporter element, encoding a polypeptide that is readily detected due to a colorimetric, fluorescent, luminescent, or enzymatic property, and an enhancer/promoter element, which confers specificity to the expression of the reporter gene. Reporter elements include, without limitation, luciferase, beta lactamase, green fluorescent protein, blue fluorescent protein, chloramphenicol acetyltransferase (CAT), beta galactosidase, and alkaline phosphatase. Enhancer/promoter elements include, for example, those responsive to NFAT, p53, TGF-beta, or any other signaling pathway or stimulus for which a responsive promoter/enhancer is known.

### NFkB

One commercially available reporter gene is pNFkB, a luciferase reporter gene that produces light when NFkB is activated (Stratagene, La Jolla, CA). This reporter gene can be transfected, either stably or transiently, into a cell line of interest (i.e., A549 human lung carcinoma cells). To generate a stably transfected cell line, the pNFkB plasmid DNA is mixed with a G418 resistance plasmid and a transfection reagent (e.g., lipofectamine, Life Technologies, Inc., Rockville, MD), and added to a 10 cm petri dish containing A549 or other cells attached to the dish at approximately 40% confluency. The plate with DNA/lipofectamine is incubated for 4-16 hours at 37°C with 5% CO₂. The medium is removed, the cells are washed twice with serum-free medium, and then medium with 10% fetal bovine serum (FBS) is added. G418 is added at a dose (approximately 700 µg/mL) that is just sufficient to kill all cells in a mock transfection (i.e., a transfection without a G418-resistance plasmid). The cells are incubated at 37°C with 5% CO₂ for 2 weeks, with medium changes every three days. Stably-transfected clones will have grown as small colonies by the end of two weeks; these clones are separated by ring-cloning and propagated separately. G418-resistant clones are screened for the reporter gene of interest by measuring light output from cell lysates with or without transiently transfected NFkB present. Once a clone is identified with stably-transfected pNFkB, it is used for screening by plating the cells in the assay plate.

For a transient transfection assay, the pNFkB plasmid DNA is mixed with a transfection reagent, added to a 10 cm petri dish containing, for example, A549 cells attached to the dish at approximately 70% confluency. The plate with DNA/lipofectamine is incubated for 4-16 hours at 37°C with 5% CO₂. The medium is removed, the cells are washed twice with serum-free medium, and then medium with 10% FBS is added. The cells are incubated at 37°C with 5% CO₂ for 24 hours, then plated into assay plates for screening.

The reporter gene can be introduced into the cells using other techniques, including, without limitation, viral or retroviral infection, bolistic injection, and cellular uptake of naked DNA. One in the art will recognize that any method of introduction of the reporter gene into the cells to be assayed will be compatible with the screening methods described herein.

Once the cells with the reporter gene are available, they are seeded in assay plates (96 well, 384 well, etc) with a pipette, multichannel pipette, 384 well Multidrop platefiller (Labsystems, Franklin, MA), or other liquid handling device. Compounds are added to form combinations by one of several methods. After 4-72 hours, the medium is removed, the cells are washed twice with HBSS, a lysis buffer is added (see Stockwell et al., J. Amer. Chem. Soc. 1999, 121:10662-10663), ATP/luciferin added and luminescence is measured on a platereader or luminometer (e.g. LJL BioSystems Inc., Analyst AD, Sunnyvale, CA).

### Fluorescence Resonance Energy Transfer Assays

In another example, fluorescence resonance energy transfer (FRET) is used to detect and measure the interaction of two proteins of interest. In this example, the first and second proteins are fused with green fluorescent protein (GFP) and blue fluorescent protein (BFP), respectively, using standard molecular biological methods. The DNA constructs encoding the two fusion proteins are co-expressed in mammalian cells, yeast, worms, or other cell or organism using transfection techniques described above or other comparable methods. Combinations of compounds are added. The plate is placed on a platereader and fluorescence is measured as follows. The donor fluorophore (i.e., BFP) is excited and the emission of the acceptor fluorophore (i.e., GFP) is measured. Increased proximity of the two proteins will result in an increase in emission of the acceptor fluorophore. Thus, a combination of compounds that causes the two proteins of interest to be near each other is identified by an increase in fluorescence of the acceptor fluorophore.

For example, expression vectors containing Smad2 and Smad4 are obtained. The cDNA for GFP is fused to the 5' end of Smad2 and the cDNA for BFP is fused to the 5' end of Smad4. These expression vectors are transfected into mammalian cells stably or transiently, cells are treated with combinations of compounds, and the plate is irradiated with light that excites BFP but not GFP. Fluorescence of GFP (e.g., 512 nm light) is measured and combinations of compounds that cause an increase in light emission at this wavelength are identified. Such combinations are causing Smad2 and Smad4 to localize near each other, and may be activating TGF-beta signaling, arid therefore may be useful for treating cancer chemotherapy mucositis, and autoimmune diseases.

### Fluorescent Calcium Indicator Dyes

Another readout of a change in a property of a test element utilizes fluorescent calcium indicator dyes, such as fluo-3 (Molecular Probes, Eugene WA; http://www.molecularprobes.com), fura-2, and indo-1. Fluo-3 is essentially nonfluorescent unless bound to Ca²⁺ and exhibits a quantum yield at saturating Ca²⁺ of ~0.14. The intact acetoxymethyl ester derivative of fluo-3 AM (fluo-3 AM) is therefore also nonfluorescent. The green-fluorescent emission (-525 nm) of Ca²⁺-bound fluo-3 is conventionally detected using optical filter sets designed for fluorescein (FITC). According to the manufacturer, fluo-3 exhibits an at least 100-fold Ca²⁺-dependent fluorescence enhancement.

Cells are seeded in wells, fluo-3 is added to the cells following the manufacturer's instructions, compounds are added, and fluorescence is measured using a plate reader. Combinations of compounds that result in an increase in fluorescence (but are not themselves fluorescent) are thus causing an increase in the concentration of intracellular calcium.

### Fluorescence Microscopy

Another assay uses conventional fluorescence microscopy to detect a change in the level or localization of fluorescence in cells contacted with combinations of compounds. In one example, a stably transfected cell line expressing a GFP tagged Smad2 is used. Cells are seeded, compounds are added and incubated for 1 hour, and a fluorescence microscope with an automated stage is used to image the cells in each well. Combinations of compounds that cause a change in the localization of the tagged protein are identified. For example, combinations that cause GFP-Smad2 to translocate from outside of the nucleus to the interior of the nucleus can be identified in this manner. These combinations may be activating TGF-beta signaling and, thus, may be useful for treating cancer, autoimmune diseases, and mucositis.

### Expression Profiling with cDNA Arrays

Another assay for the detection of compounds that, together, produce an alteration in a test element is expression profiling. In this example, cells are seeded, combinations of compounds are added, and the cells are incubated for 2-24 hours. PolyA RNA is harvested from each well using standard methods. The RNA is reverse transcribed into cDNA using standard methods, with the exception that a fluorescent dye (e.g., Cy3-dUTP) is incorporated during the reverse transcription. The Cy3-labeled cDNA is mixed with Cy5-labeled cDNA from untreated cells and hybridized to a DNA microarray, (e.g., a DNA microarray commercially available from Affymetrix, Santa Clara, CA, or Incyte, Palo Alto, CA reviewed in Nature Genet. Suppl. 21, Jan 1999 (hereby incorporated by reference)). The relative level of Cy3 and Cy5 fluorescence at each spot in the microarray indicates which there has been a change in the expression of each gene. The method is used to identify combinations that cause a desired change in gene expression, such as reversion of a disease gene expression profile back to a healthy profile. Alternatively, the expression profile caused by a given signaling molecule, such as insulin, is determined, and combinations of compounds are found that cause the insulin profile to appear, indicating these combinations mimic the effects of insulin.

### Whole Organisms

Another bioassay that is compatible with the screening assays described herein utilizes a whole animal. In one example, the nematode *C. elegans* is placed into individual wells (preferably with more than one nematode per well), and the activity of compounds is detected by detecting a change in a property of the organism. For example, the nematode can be engineered to express green fluorescent protein at a specific stage of the life cycle, or only during the dauer state. An automated microscopy system is used to image the nematodes in each well and measure green fluorescent protein, or detect morphological changes in the worms caused by particular combinations of compounds.

Another whole animal assay uses large animals, such as nude mice, that have tumors on or near the skin surface. The combinations of compounds can be mixtures in DMSO that are rubbed into the skin, penetrate the skin, and reach the tumor. Alternatively, the compounds can be administered intravenously, intramuscularly, or orally. Other whole-organism methods of detecting activity could include using small tadpoles derived from fertilized *Xenopus* oocytes that develop in defined medium, organotypic cultures (explants from mice or other animals) in which the organ can be cultured for a period of time in a defined medium, and eggs (fertilized or unfertilized) from a variety of animals. Another assay measures the tension of cardiac tissue or muscle tissue stretched between two springs; compound combinations that modulate contraction would result in increased or decreased in the tension on those springs.

### Labeling of Compounds

Although some assays can be conducted without any of the combined entities being labeled, in some embodiments one or more of the combined entities is labeled so that the effect of the combination on the test element can be detected or measured. Any of a wide variety of known labels can be used, e.g., techniques that have been used widely in biochemistry for protein affinity chromatography using biotin-streptavidin interactions or hexahistidine tagged proteins (Janknect et al., Proc. Natl. Acad. Sci. USA 1991, 88:8972; Wilcheck et al Methods in Enzymology, Wilcheck, M; Bayer, E.A. Eds. Academic Press Inc. San Diego, 1990; pp. 123-129).

### Combining of the Molecules

The methods of the invention can use existing robotics systems, 96-well, 384-well, 1536-well or other high density stock plates and 96-, 384-, or 1536-well or other high density assay plates, with which it is possible to screen up to 150,000 compounds or more per week. The automation of this technology can be adapted according to the invention to screen combinations of molecules. The methods of the invention may also use microfluidics systems made either by conventional photolithography or by unconventional methods (such as soft lithography or near-field optical lithography) to miniaturize the process. The methods of the invention may also use ink-jet printing or compound printing technologies. Additionally, the methods of the invention may use trained technician labor to achieve the same results and throughput as the robotics systems. The combinations of compounds may be made prior to contact with the test element, or they may be in situ in the presence of the test element. These plating methods are described in more detail below.

### Manual Plating

Compounds may be plated (i.e., added to the cells to be tested) manually. In one example, purified chemical compounds are manually combined and tested in a 7X7 combinatorial array. The compounds, which in this case include seven compounds, are in a stock plate. The compounds are combined in an assay plate. The operator plates a first compound (or plurality of compounds) from a well of the stock plate in one row of the assay plate and then plates one column in the assay plate. This is repeated with a second well from the stock plate, only the plating in the assay plate is one row over and one column over from those into which the first compound was plated. This process is repeated until the full set of combinations has been plated.

### Robotic Plating

There are numerous methods for adding compounds to wells to form combinatorial arrays, and one skilled in the art will recognize that the following examples are for illustrative purposes and are in no way limiting.

In one example of robotic plating, a robotic liquid transfer systems is used. Transfer systems are commercially available from, for example, Beckman Coulter (Fullerton, CA), Tecan (Research Triangle Park, NC) or Zymark (Hopkinton, MA). The robotic system plates specific volumes of the first set of compounds into each well in a given row, such that row 1 will have the same compound, row 2 will have the same compound, etc. Then, the liquid transfer device plates the same set of compounds along the columns such that each column will receive the same compound (although different columns will have different compounds). Transfer systems can be adapted for transferring small volumes (e.g., 1 nL).

Another method for effective transfer utilizes ink-jet printing technology (Gordon et al., J. Med. Chem. 1994, 13:1385-1401; Lemmo et al., Curr. Opin. Biotechnol. 1998, 9:615-617). Ink-jet printers draw from a plurality of vessels containing test compounds; each compound from a source well is printed out or injected onto the surface in each individual row and column for each compound. As describe above, the next compound is printed out onto the next row and the next column, and this is iterated until the entire grid is plated with combinations of compounds.

Yet another method for adding compounds to an assay plate utilizes a microarray spotter as developed by Patrick Brown at Stanford University for spotting DNA. This device uses an eight-quill pen printing head and eight linear quillheads that are dipped in a stock plate and printed along every row. Subsequently, either the plate is rotated ninety degrees or the printing head is rotated ninety degrees; the printhead then prints along the columns. One could vary the size of the print head using, for example, two, four or sixteen printhead for standard 384 well plates, and higher numbers for higher density plates.

Yet another method for adding compounds to an assay plate uses a commercially available instrument called the Hydra (Robbins Scientific, Sunnyvale, CA), which can be equipped with 384 separate syringes that are capable of dropping a known volume from a standard stock plate of compounds.

An alternate method for the plating of test compounds uses microfluidics systems such as those commercially available from Caliper Technologies (Mountain View, CA) and directly applying that system to creating an array that would create this combination. In this case, arrays of combinations at the microscale are created using capillary flow to distribute the compound solutions to the intersection points on a matrix.

### Alternative Plating Methods

One in the art will recognize that any plate configuration can be adapted to the screening methods of the invention. For example, a 16 X 16 square plate would have 256 wells instead of the 384 wells in a 16 X 24 plate. This would allow one to adapt any liquid addition system in which liquid is only added along the rows or only along the columns because one could simply rotate the square plate ninety degrees and allow addition in the other direction. One would also incorporate into this design, a square plate holder that would have the dimensions or footprint of a standard 96 well or 384 well microtiter plate so that the adapted microsquare plate would fit within any existing plate liquid handling system.

Another method of providing the compounds or elements to be tested in combination is to provide them in solid form rather than liquid form, e.g., as small amounts of dry powder. Thus, two dry components (or one wet and one dry component) can be mixed and then the combination added to the cells in the medium (in which the combined entities are soluble). Solid compounds include beads from a combinatorial synthesis on which a different compound is added. For example, beads could be added with a bead picker. In one example, the beads are magnetic and added using a magnet.

In the high throughput assays of the robotics application of the invention, each well must be spatially addressable independently, and it is preferably possible to withdraw both large (up to 100 µL) and small (down to 1 nL) of each compound from a stock plate. An exemplary robotic platform for performing combinatorial screening assays of the invention is described below.

A two station robotic platform is created. The first station harbors a simple XYZ robotic arm with an attached pin transfer device such as is available through VWR (cat#62409-608). A stock plate and an assay plate enter the station and the robotic arm drops the pins into the stock plate and transfers these pins into the assay plate, thereby delivering 1-1000 nL, depending on pin size (most typically, 50 nL are delivered). Different pin devices allow transfer of different combinations of compounds, as described above in the example. The second station of the robot is a piezo electric dispensor, capable of withdrawing large volumes (up to 10 microliters) from a stock well of a single compound and then dispensing small volumes into each well of an assay plate. For example, the Ivek Digispense 2000 system (http://www.ivek.com/digi2000.html) has a resolution of 10 nL and should be sufficient for this purpose.

### Non-Compound Combination Reagents

If a combination reagent which is present across the entire plate is not a compound (i.e., if it is radiation of some form), then the plate that contains the cells and the added compound(s) can be passed by that source of radiation, thereby completing the forming of the combination. Other forms of non-compound combination reagents include, for example, hyperbaric pressure and heat.

Some non-compound reagents can be varied in a manner similar to the addition of a compound. For example, to alter pH, different amounts of base or acid are added to the wells. Similarly, ions can be added using any method that is applicable for the addition of compounds.

### Library Size

For small chemical libraries (<1000 compounds), all binary (up to one half million combinations) and tertiary combinations (up to several hundred million combinations) can be tested using the systems described herein.

The power of combinations quickly generates effective libraries to identify multivariable therapeutics. The size of multivariable therapeutic libraries quickly rivals and then expands beyond any conventionally available diverse or non-diverse libraries. A library of 200 compounds in pair-wise combinations generates a multivariable therapeutic library of 19,900 combinations, larger in size than the natural product library recently used to identify a novel compound affecting cell division (Mayer et al. Science, 1999, 286, 971-974). A library of 300 compounds in 3 way combinations generates approximately 4.5 million distinct combinations of entities for a multivariable therapeutic library, larger than the largest currently generated combinatorial chemistry libraries and with potentially greater diversity.

### Activity Rank-Order Screen

A diverse library may initially be tested individually using standard methodologies. The compounds are screened in a biological assay and ranked by activity. The most active compounds (e.g., the twenty most active or the thousand most active, depending on the amount of combinatorial space to be assayed) in the library are then tested pair-wise and in three-way combinations. If desired, these combinations can be again ranked by activity and the process iterated for four-way combinations, five-way combinations, etc., until a desired number of effective combinations are identified.

### Genetic Algorithms

The use of genetic algorithms provides another method for identifying combinations of agents with a desired biological activity that is distinct from the single agents themselves. In this method each single agent is assigned a unique barcode, which can be any series of numbers, letters, or other symbols. In a preferred mode of embodiment, the barcode is a binary code (zeros and ones). A specified number (N, the "population size") of pair-wise combinations are selected (randomly or based on some other characteristic) from the total pool of possible pair-wise combinations and the activity of each of these combinations is determined. The X most active members of the population are selected (where X is less than N). N new combinations are formed from the X active combinations using a series of operators which include (i) replication (the original combination is simply selected again) (ii) mutation (one bit of the barcode is mutated to a different value), and (iii) recombination (two barcodes are each split at some middle point and the opposing ends are joined to create two new hybrid barcodes. This process of selection, amplification, mutation, and recombination is repeated over numerous cycles, and the activity of each combination of agents determined at the end of each cycle. This provides a method of testing a limited number of combinations, while still allowing for the discovery of highly effective combinations of agents.

A method for optimizing the parameters of the genetic algorithm (or other algorithm) is as follows: C combinations of N agents are selected for study. The activity of each member of the full set of (N!)/((N-C)!(C!)) combinations is determined. Furthermore, all lower order combinations may be determined as well. With this full data set of activities, it is possible to evaluate the success rate and efficiency of various algorithms, or various permutations of a single algorithm without doing additional "wet" benchwork. That is, all strategies for finding active combinations can be compared "in silico" and their relative performance compared.

### Stock Solutions

Two types of stock solutions are maintained. Each compound is deposited in both stock formats. The first stock solution format consists of compounds dissolved in 100 microliters of dimethylsulfoxide (DMSO) at a final concentration of 4 mM and stored in 384 well polypropylene plates (Matrix Technologies). The second type of stock solution consists of compounds dissolved in 1500 microliters of DMSO at a final concentration of 4 mM and stored in deep-well 96 well polypropylene plates. Multiple copies of each type of plate are made. One copy of the stock plates is stored at -20°C for routine use, and backup copies are stored at -80°C for long-term storage.

### Assay Plates

Three types of assay plates are used. The size of the pins in the pin transfer devices described above is adapted to accommodate each size assay plate.
1) Commercial 384 well plates (e.g. NalgeNunc white opaque polystyrene cell culture treated sterile plates with lid, cat# 164610)
2) Commercial 1536 well plates (e.g. Greiner or Corning)
3) Custom prepared 1536 or 6144 well plates (made from polydimethysiloxane, Dow Coming) and delran molds, and Omni trays.

### Software to Manage Compound Additions

Microsoft Visual Basic or programming language is used, using conventional programming techniques, to write software to operate the instruments described above. The software will permit the instrument to read the barcode of a stock plate or assay plate, track the location of plates on the assay deck, and transfer the appropriate volume of the correct compound into the correct assay well. Thus all combinations to be screened are determined or selected beforehand, and the instrument carries out the combination screening in an automated format, requiring only simple operator steps, such as placing specified plates onto the assay deck and removing specified plates from the assay deck to an incubator.

### Barcode Reader and Printer

A barcode printer is used, using standard techniques, to generate a unique identification number for each plate, print the barcode on a label, and stamp the label on the assay or stock plate. Software records the identity of each compound in each well of each assay plate and stock plate. A barcode reader linked to the assay deck scans each plate as it enters and leaves the assay deck.

The following examples are provided for illustrative purposes, and are not meant to be limiting in any way.

### Example 1

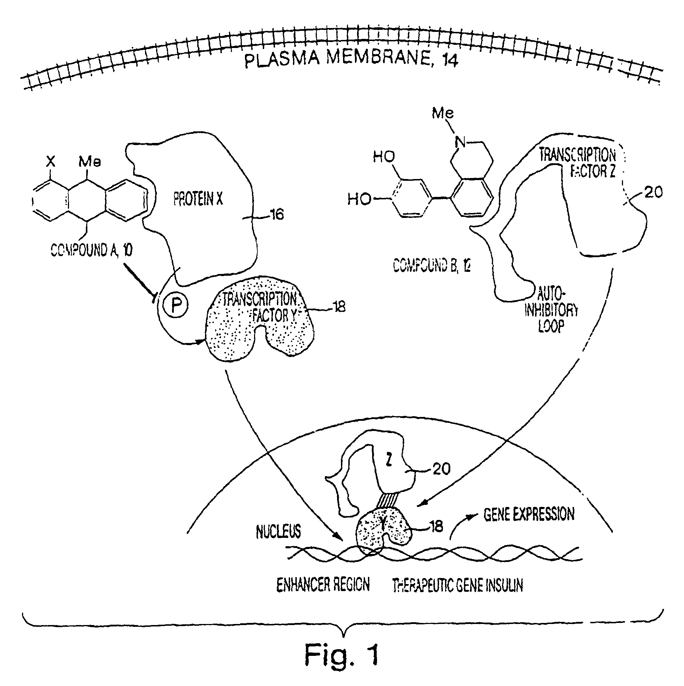

Fig. 1 is a conceptual diagram demonstrating how two different reagents could act synergistically inside of a cell, where the reagents bind to different targets within the same cell. In this figure, compound A **10** and compound B **12** cross the plasma membrane **14** and diffuse freely into the cytosolic region of a mammalian cell. Compound A binds to protein X **16,** which is a kinase, inhibiting the activity of this kinase. Kinase X normally inactivates transcription factor Y **18** by adding a phosphate group to Y. Once compound A has inhibited kinase X, transcription factor Y is activated, and Y translocates into the nucleus, binding tightly to DNA in the enhancer region of a therapeutic gene, such as insulin. However transcription factor Y is unable to activate expression of insulin without the presence of a second transcription factor Z **20**. However, in the figure, compound B binds to transcription factor Z, removing an autoinhibitory loop on this transcription factor, thereby causing this transcription factor Z to translocate into the nucleus, and bind to transcription factor Y. Y and Z together, but neither alone, allow activation of expression of the therapeutic gene, insulin.

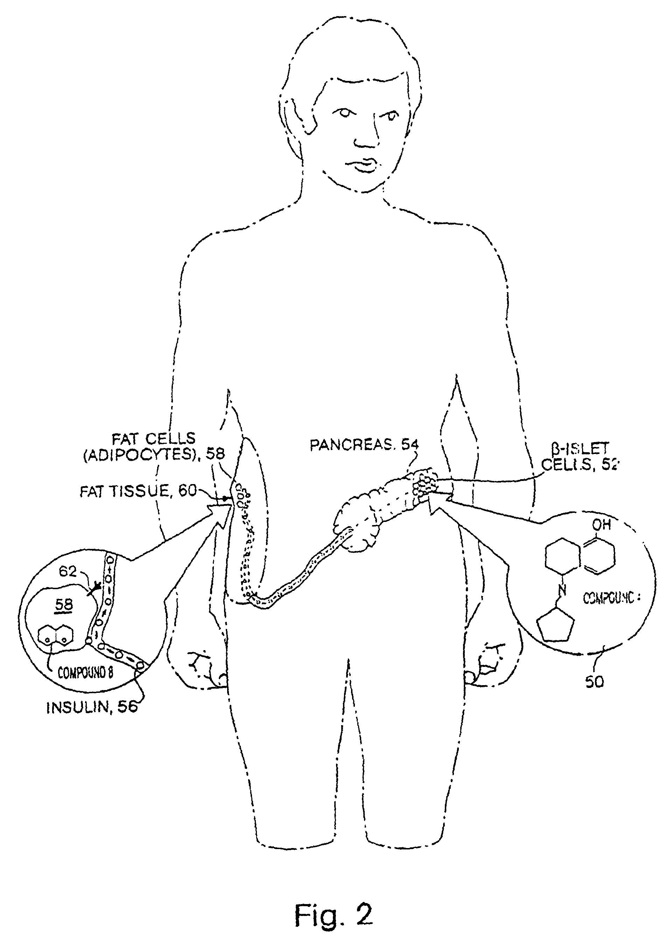

Fig. 2 is a conceptual diagram demonstrating how two different reagents could act synergistically inside of an organism, where the reagents bind to targets in different cells or tissues. In this figure compound A **50** diffuses into beta islet cells **52** of the pancreas **54**. Compound A causes a therapeutic gene encoding insulin **56** to be expressed in these cells. However, insulin is ineffective without the presence of the insulin receptor on target adipocytes in fat tissue. Meanwhile, compound B diffuses into adipocytes **58** in fat tissue **60** and turns on expression of the insulin receptor **62** in these cells. A and B together, but neither one alone, allow insulin activity to be restored in this individual.

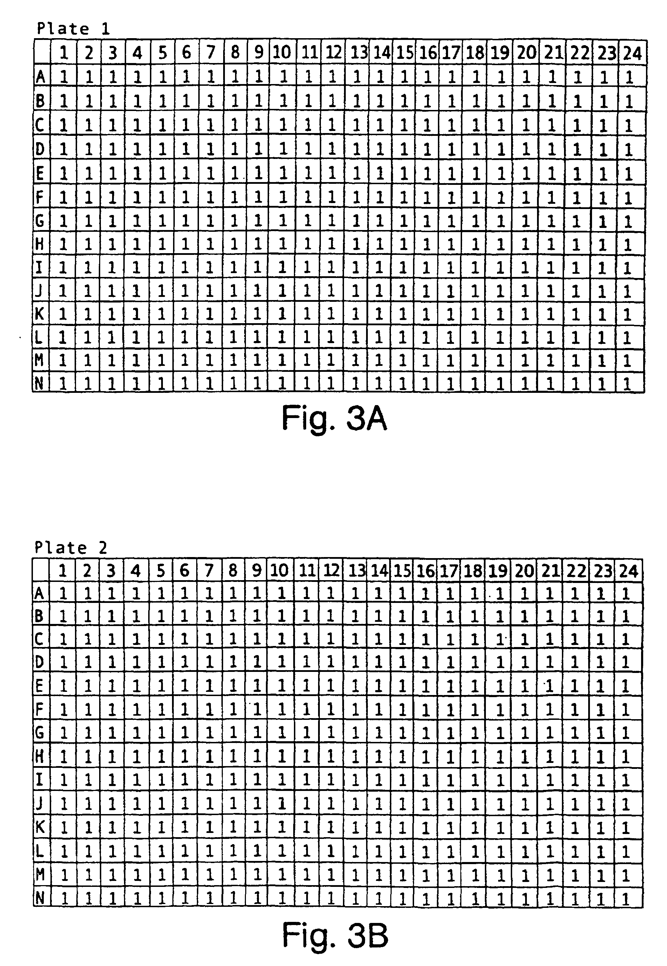

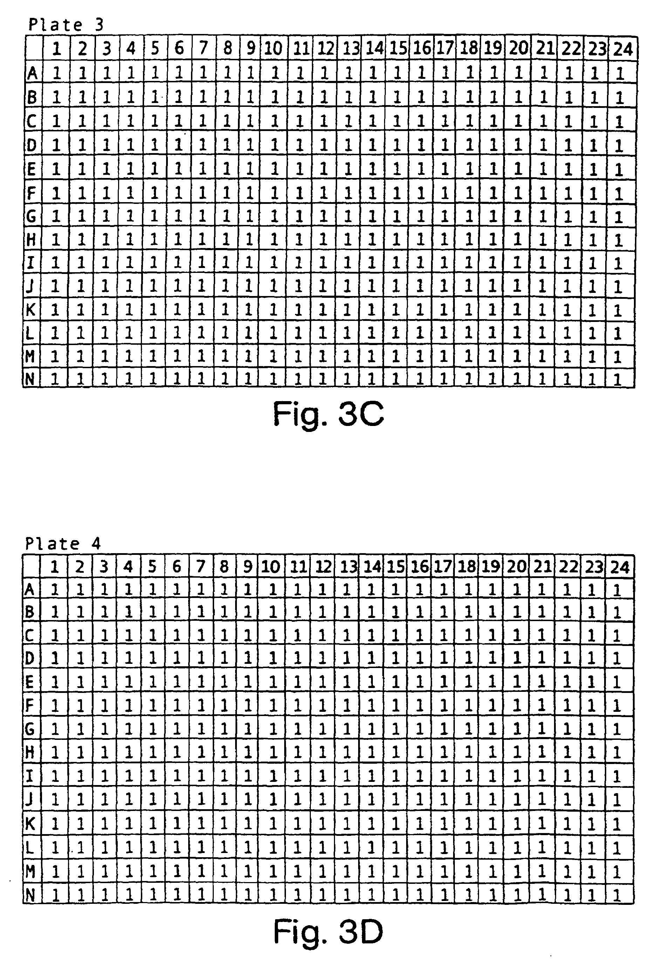

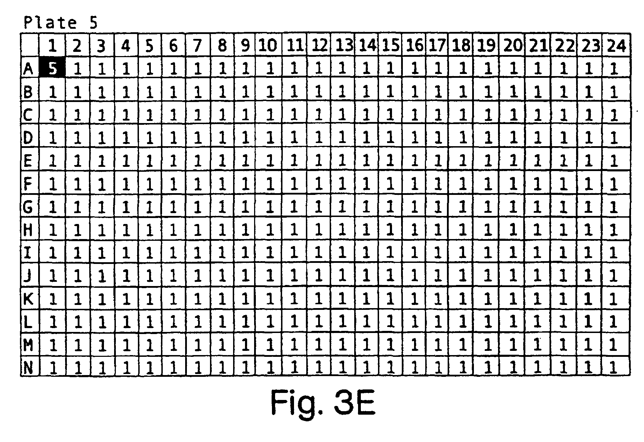

Fig. 3 is an example of the experimental data one would obtain in a combinatorial screen of the sort we describe in this patent application. The results from five different 384 well plates are shown. The results are shown in plate format, where there are 16 rows labeled A through P, and 24 columns, labeled 1 through 24. The level of activity is shown in each well as a number, where 1 indicates basal activity (no effect) and 5 indicates an active combination. Plate one shows the activity of compounds 1-384, when tested at 4 µg/mL in a bromodeoxyuridine cytoblot assay for cell-cycle arresting activity in A549 human carcinoma cells (described below). Plate two shows the activity of compounds 1-384 when tested at 2 µg/mL in same assay. Plate three shows the activity of compounds 385-768, when tested at 4 µg/mL, in the same assay. Plate four shows the activity of 385-768, when tested at 2 µg/mL, in the same assay. Note that in plates 1-4, none of the compounds shows any activity. Plate five shows the activity of 384 pair-wise combinations of compounds 1-768, when tested at 2 µg/mL (i.e., compounds 1-384 and 385-768 were both added simultaneously to the assay plate at 2 µg/mL each compound, creating 384 different random pair-wise combinations). Note that well A1 shows activity. This means that compound 1 (in well A1 of the plate with compounds 1-384) by itself had no activity and compound 385 (in well A1 of the plate with compounds 385-768) by itself had no activity but together compound 1 and 385 synergize to create an active combination.

### Example 2

### General Methods

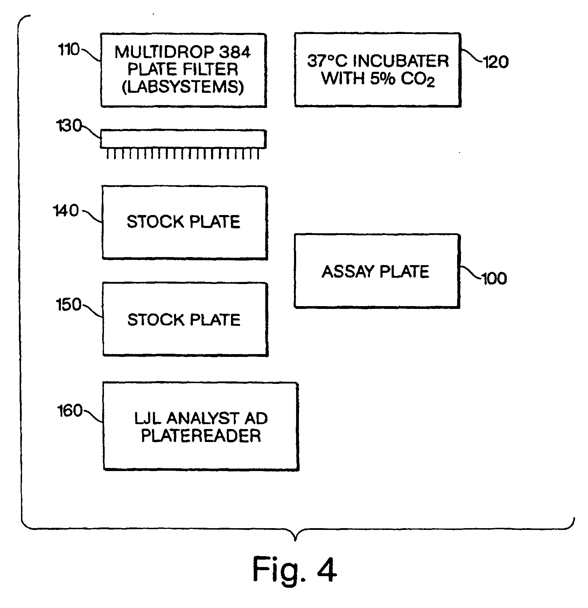

Fig. 4 is an illustration of a method for performing combinatorial screening using currently commercially available technology. Human A549 lung carcinoma cells are obtained from the American Type Culture Collection (ATCC, catalog number CCL185) and cultured at 37°C with 5% CO₂ in Dulbecco's Modified Eagle Medium (DMEM) with 10% FBS, 100 units/mL penicillin G sodium, 100 µg/mL streptomycin sulfate, and 2 mM glutamate (GibcoBRL, Rockville, MD) (referred to as 10% medium). Four thousand cells are seeded in each well of a white, opaque 384-well plate **100** (Nalge Nunc International, Rochester, NY) using a Multidrop 384 liquid dispenser **110** (Labsystems Microplate Instrumentation Division, Franklin, MA). The cells are seeded in 40 µL of 10% FBS-containing medium.

After 16 hours at 37 C with 5% CO₂, 10 µL of a 50 µM stock of a compound of interest in 10% medium is added to each well, bringing the total well volume to 40 µL and the final concentration of this compound to 10 µM. Either prior to, immediately afterwards, or several hours or days later, a second set of compounds is added by dipping small pins on a pin array **130** into each well of a stock plate **140** or **150** and then into each well of an assay plate **100.** Matrix Technologies Pin Transfer device **130** (384 or 96 pins, will suffice (catalog numbers 350500130 and 350510203). This two-step process allows for the testing of one specific compound against a large number of other compounds in many pair-wise combinations. It is necessary to also have a plate where the set of pin-transferred compounds is tested in the absence of the original compound (the one present in all wells) to determine whether a novel property has been achieved with the combination.

A different method can also be used to provide combinations of compounds for contacting with the test element. For example, instead of keeping one compound fixed throughout the pair-wise combinations, as described above, it is possible to pin transfer a set of compounds in such a way that all pairwise combinations of that set are achieved. A set of 16 compounds is pin transferred from stock plate **140** to the 16 rows of the 384 well assay plate **100**. The same set of 16 compounds is then transferred to 16 columns of the same assay plate, providing a 256 well matrix with different pairwise combinations.

In each of the foregoing examples (or analogous versions), once the desired compounds have all been added, the assay plate containing A549 cells and compounds are incubated for 48 hours at 37°C with 5% carbon dioxide in incubator **120**. At the end of this time, 10 µL of BrdU is added, using a Multidrop 384 **110**, from a 50 µM stock in medium (prepared from a 10 mM stock in PBS, pH 7.4) to a final concentration of 10 µM BrdU. The cells are incubated for an additional 12-16 hours in incubator (**120**) at 37°C with 5% CO₂. The supernatant is removed from each well with a 24-channel wand (V&P Scientific), used throughout the protocol for aspiration, attached to a house vacuum source. The cells are fixed with 50 µL of cold (4 C) 70% ethanol/30% PBS, incubated for one hour on ice, washed with 90 µL of cold (4 C) PBS, and then 25 µL of 2M HCl / 0.5% Tween20 ddH2O is added. The cells are incubated at room temperature for 20 minutes, then washed with 90 µL of 10% 2M NaOH / 90% Hank's Balanced Salt Solution (HBSS, GibcoBRL), twice with 90 µL of HBSS, and once with 75 µL of PBSTB (PBS, 0.1% Tween 20 (Sigma), 0.5% bovine serum albumin (Sigma-Aldrich, St. Louis, MO). Then 20 µL of antibody solution is added containing 0.5 µg/mL mouse anti-BrdU antibody (1:1000 dilution of stock, (BD Biosciences-PharMingen San Diego, CA) and 1:2000 dilution of anti-mouse Ig antibody conjugated to HRP (Amersham Pharmacia Biotech Inc., Piscataway , NJ) in PBSTB. The cells are incubated for one hour at room temperature, washed twice with 90 µL PBS and then 20 µL HRP substrate solution is added to each well. The HRP substrate solution is obtained by mixing equal volumes of solutions 1 and 2 from the Amersham ECL detection kit. The plate is placed into an LJL Biosystems Inc. (Sunnyvale, CA) Analyst AD platereader **160** and luminescence is detected in each well for 0.3 seconds. The activity of combinations are then compared to the activity of the single agents alone at both the original concentration and at N times the original concentration, where N is equal to the number of active compounds found in the screen. If a combination shows an activity that is greater than the activity of the each of the single agents alone at both the original concentration and N times the original concentration, then a synergistic effect has been observed. Even if synergy is not observed, a combination may have advantageous properties (e.g., reduced side effects) relative to the individual components that make up the combination.

The foregoing method is readily modified such that two different compounds are added in the first step, allowing the testing of three-way combinations. Similarly, three compounds can be added to allow testing of four-way combinations, etc. Using such an approach, higher order combinations of compounds are tested.

The identification of a combination of compounds that inhibit proliferation is described below. This description is for illustrative purposes, and should is not limiting in any way.

Seven compounds were tested alone and in all 21 possible pair-wise combinations in the BrdU cytoblot assay (see below) for their effect on cell cycle progression. The seven compounds (podophyllotoxin, paclitaxel, quinacrine, bepridil, dipyridamole, promethazine, and agroclavine; each purchased from Sigma Aldrich Corp., St. Louis, MO) were weighed into one dram glass vials and dissolved in dimethylsulfoxide to create 4 mg/mL stock solutions. Six thousand A549 lung carcinoma cells were seeded in each well of a 384 white opaque NalgeNunc cell culture-treated plate (cat# 164610) in 30 µL of 10% medium (Dulbecco's Modified Eagle Medium containing 10% fetal bovine serum, 100 units/mL penicillin G sodium, 100 µg/mL streptomycin sulfate, and 2 mM glutamine, all obtained from Life Technologies). Each compound was diluted to four times the final assay concentration (final assay concentrations were 0.25% DMSO, 240 nM podophyllotoxin, 60 nM paclitaxel, 420 nM quinacrine, 25 µM bepridil, 400 nM dipyridamole, 25 µM promethazine, 840 nM agroclavine) in 10% medium. Fifteen microliters of each 4X stock of compound in medium was added to one row and one column of an 8 column and 8 row square (the eighth lane containing only the vehicle DMSO), such that all possible binary combinations of the 7 compounds were tested, as well as the single agents themselves. The cells were incubated at 37°C with 5% carbon dioxide for 46 hours. BrdU was added to a final concentration of 10 µM by adding 15 µL of a 50 µM solution ofBrdU in 10% medium. The cells were incubated overnight at 37°C with 5% carbon dioxide.

After 16 hours the medium was aspirated from each well with a 24 channel wand (V&P Scientific), used throughout the protocol, attached to a house vacuum source. Fifty microliters of 70% ethanol/ 30% phosphate buffered saline (4°C) was added to each well with a Multidrop 384 plate filler (Labsystems), used for all subsequent liquid addition steps. The plate was incubated for one hour at room temperature, then the wells were aspirated, and 25 µL of 2 M HCl with 0.5% Tween 20 was added to each well. The plate was incubated for 20 minutes at room temperature. Twenty five microliters of 2 M NaOH was then added to each well. The liquid in each well was aspirated and the wells were washed twice with 75 µL of Hank's Balanced Salt Solution. The wells were washed again with 75 µL of PBSTB (phosphate buffered saline with 0.5% bovine serum albumin and 0.1% Tween 20). Twenty microliters of antibody solution was added to each well (containing 0.5 µg/mL anti-BrdU antibody (PharMingen) and 1:2000 dilution of anti-mouse Ig-HRP (Amersham). The plate was incubated with the antibody solution for one hour at room temperature, then the antibody solution was aspirated off and each well was washed once with phosphate buffered saline. Finally, 20 µL of ECL detection reagent was added to each well (an equal mixture of solutions one and two from Amersham's ECL detection reagents). The luminescence in each well was read on an LJL Analyst platereader with 0.2 seconds integration time per well. The experiment was repeated in two plates and each pair-wise combination was tested in a total of sixteen replicate experiments. The data shown in the table depict the mean antiproliferative activity of each combination of compounds. Five statistically significant (p < 0.001) combinations are highlighted. All activity is normalized to a set of wells containing cells that did not receive any treatment. Thus, a value of one represents an inactive substance and a value greater than one indicates some level of antiproliferative activity.

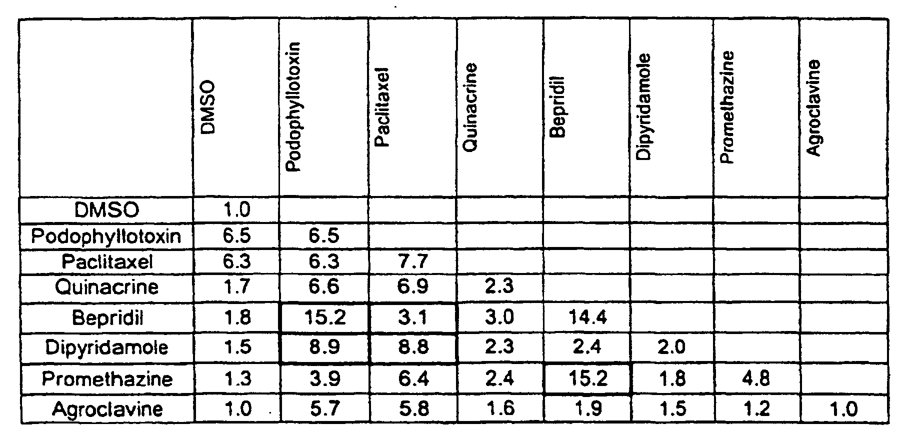

The chart shows five combinations of existing FDA-approved drugs with antiproliferative activity that is distinct from that of the individual components. Podophyllotoxin and paclitaxel are both microtubule stabilizers that arrest cells in mitosis, dipyridamole is an anti-platelet agent, bepridil is a calcium channel blocker, and promethazine is an H1 histamine receptor antagonist and is also used as a CNS depressant and anticholinergic agent. Dipyridamole is generally considered to have a relatively high safety profile as a human therapeutic, particularly compared to the toxic side effects of paclitaxel and podophyllotoxin. Thus, in this assay, dipyridamole enhances the antiproliferative effect of both paclitaxel and podophyllotoxin on human lung cancer cells. Furthermore, bepridil enhances the effects of podophyllotoxin but inhibits the effect of paclitaxel. This result would not have been predicted a priori and highlights the importance of empirical high throughput testing of combinations to observe unexpected interactions among drugs. For example, bepridil and promethazine, neither of which is used as an antiproliferative agent in current therapeutic indications, combine to strongly inhibit the proliferation of lung cancer cells.

### Other Embodiments

All publications and patents mentioned in the above specification are herein incorporated by reference. Various modifications and variations of the described method and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the field of drug discovery or related fields are intended to be within the scope of the invention.

## Claims

1. A method for screening combinations of compounds for identifying combinations of compounds that cause an effect that is different from the effect of a compound of the combination by itself, said method comprising the steps of
(a) providing whole cells of a whole cell assay,
(b) contacting said whole cells with at least 100 compounds under conditions that ensure that each compound/whole cell contacting is segregated from the others, and
(c) detecting or measuring effects displayed by the compounds on the whole cells,
**characterized in that** said method comprises the additional following steps of:
(d) selecting compounds that cause a change in said effect displayed on the whole cells relative to said effect of said whole cells not contacted with said compounds,
(e) creating an array of at least 49 unique two-entity or higher order combinations from the identified compounds,
(f) contacting said array of combinations of compounds with whole cells of the whole cell assay under conditions that ensure that each compound combination/whole cell contacting is segregated from the others,
(g) detecting or measuring an effect displayed by the combinations of compounds on the whole cells of step (f), and
(h) identifying combinations of compounds that cause an effect of step (g) that is different from the effect of a compound of the combination by itself

2. The method of claim 1, wherein step (e) comprises creating an array of at least 200 unique combinations of two or more compounds.

3. The method of claim 2, wherein step (e) comprises creating an array of at least 400 unique combinations of two or more compounds.

4. The method of claim 3, wherein step (e) comprises creating an array of at least 1540 unique combinations of two or more compounds.

5. The method of claim 1, wherein said detecting step (g) is performed by a cytoblot assay.

6. The method of claim 1, wherein said detecting step (g) is performed by a reporter gene assay.

7. The method of claim 1, wherein said detecting step (g) is performed by a fluorescence resonance energy transfer assay.

8. The method of claim 1, wherein said detecting step (g) is performed by detecting a fluorescent calcium-binding indicator dye.

9. The method of claim 1, wherein said detecting step (g) employs fluorescence microscopy.

10. The method of claim 1, wherein detecting step (g) employs expression profiling.

11. The method of claim 1, wherein said whole cells are human cells.

12. The method of claim 1, wherein said whole cells are selected from the group consisting of a cancer cell, an immune cell, a neuron, a fibroblast, bacterial cells, and yeast cells.

13. The method of claim 1, wherein one or both of steps (e) and (g) is carried out using a robotics system.

14. The method of claim 1, wherein one or both of steps (e) and (g) is carried out using microfluidics.

15. The method of claim 1, wherein one or both of steps (e) and (g) is carried out using ink-jet printer technology.

16. The method of claim 1, wherein said compounds are selected from the group consisting of non-polymeric organic compounds, lipids, carbohydrates, peptides, inorganic compounds, and oligonucleotides.

17. The method of claim 1, wherein at least one of said compounds is employed in purified form.

18. The method of claim 17, wherein each of said compounds is employed in purified form.

19. The method of claim 1, wherein said compounds are provided as components of mixtures.

20. The method of claim 19, wherein said mixtures are natural product extracts.

21. The method of claim 1, wherein said effect is a synergistic effect.

22. The method of claim 1, wherein at least one of said compounds is a molecule with a molecular weight of less than 1500 g/mole.

23. The method of claim 22, wherein said molecule is an FDA-approved drug.

24. The method of claim 22, wherein each of said compounds is a molecule with a molecular weight of less than 1500 g/mole.

25. The method of claim 24, wherein said each of said compounds are FDA-approved drugs.

26. The method of claim 1, wherein said combinations screened for effective therapeutic combinations are two-compound combinations.

27. The method of claim 1, wherein said combinations screened for effective therapeutic combinations are three-compound combinations.

28. The method of claim 1, wherein said effect displayed on the whole cells in the whole cell assay is transport of a compound across the cell membrane, electrical potential, action potential generation, cell proliferation, cell death, cell specification, cell differentiation, cell migration, gene expression, protein levels, enzymatic activity, phosphorylation, methylation, acetylation, protein translocation to the nucleus, or ability to produce an immune response.

29. A combination of compounds selected from a pair-wise combination of bepridil and podophyllotoxin, bepridil and promethazine, dipyridamole and podophyllotoxin, or dipyridamole and paclitaxel.

30. A pharmaceutical composition comprising (i) a combination of compounds selected from a pair-wise combination of bepridil and podophyllotoxin, bepridil and promethazine, dipyridamole and podophyllotoxin, or dipyridamole and paclitaxel, and (ii) a pharmaceutically acceptable carrier.

## Patentansprüche

1. Verfahren zum Screenen von Kombinationen von Verbindungen zum Identifizieren von Kombinationen von Verbindungen, die eine Wirkung hervorrufen, die von der Wirkung einer Verbindung der Kombination an sich verschieden ist, wobei das Verfahren die Schritte umfasst:
(a) Bereitstellen von ganzen Zellen eines Ganz-Zell-Tests,
(b) Inkontaktbringen der ganzen Zellen mit mindestens 100 Verbindungen unter Bedingungen, die sicherstellen, dass jedes Inkontaktbringen von Verbindung/ganzer Zelle von den anderen getrennt ist, und
(c) Nachweisen oder Messen von Wirkungen, die durch die Verbindungen auf den ganzen Zellen entfaltet werden,
**dadurch gekennzeichnet, dass** das Verfahren die folgenden zusätzlichen Schritte umfasst:
(d) Auswählen von Verbindungen, die eine Veränderung der auf den ganzen Zellen entfalteten Wirkung im Vergleich zu der Wirkung auf die ganzen Zellen, die nicht mit den Verbindungen in Kontakt gebracht wurden, hervorrufen,
(e) Bilden eines Arrays von mindestens 49 einzigartigen Kombinationen von Zweier-Einheiten oder von höherer Ordnung aus den identifizierten Verbindungen,
(f) Inkontaktbringen des Arrays von Kombinationen von Verbindungen mit ganzen Zellen des Ganz-Zell-Tests unter Bedingungen, die sicherstellen, dass das Inkontaktbringen jeder Verbindungskombination/ganzen Zelle von den anderen getrennt ist,
(g) Nachweisen oder Messen einer Wirkung, die durch die Kombinationen von Verbindungen auf den ganzen Zellen von Schritt (f) entfaltet wird, und
(h) Identifizieren von Kombinationen von Verbindungen, die eine Wirkung des Schritts (g) hervorrufen, die von der Wirkung einer Verbindung der Kombination an sich verschieden ist.

2. Verfahren nach Anspruch 1, wobei Schritt (e) das Bilden eines Arrays aus mindestens 200 einzigartigen Kombinationen von zwei oder mehr Verbindungen enthält.

3. Verfahren nach Anspruch 2, wobei Schritt (e) das Bilden eines Arrays aus mindestens 400 einzigartigen Kombinationen von zwei oder mehr Verbindungen enthält.

4. Verfahren nach Anspruch 3, wobei Schritt (e) das Bilden eines Arrays aus mindestens 1540 einzigartigen Kombinationen von zwei oder mehr Verbindungen enthält.

5. Verfahren nach Anspruch 1, wobei der Nachweisschritt (g) mittels eines Cytoblot-Tests durchgeführt wird.

6. Verfahren nach Anspruch 1, wobei der Nachweisschritt (g) mittels eines Reportergen-Tests durchgeführt wird.

7. Verfahren nach Anspruch 1, wobei der Nachweisschritt (g) mittels eines Fluoreszenz-Resonanz-Energie-Transfer-Tests durchgeführt wird.

8. Verfahren nach Anspruch 1, wobei der Nachweisschritt (g) mittels Nachweisen eines fluoreszierenden Calcium-bindenden Indikator-Farbstoffs durchgeführt wird.

9. Verfahren nach Anspruch 1, wobei der Nachweisschritt (g) Fluoreszenzmikroskopie benutzt.

10. Verfahren nach Anspruch 1, wobei der Nachweisschritt (g) das Erstellen eines Expressionsprofils benutzt.

11. Verfahren nach Anspruch 1, wobei die ganzen Zellen humane Zellen sind.

12. Verfahren nach Anspruch 1, wobei die ganzen Zellen ausgewählt sind aus der Gruppe bestehend aus einer Krebszelle, einer Immunzelle, einem Neuron, einem Fibroblasten, Bakterienzellen und Hefezellen.

13. Verfahren nach Anspruch 1, wobei einer oder beide Schritte (e) und (g) unter Verwendung eines Robotersystems durchgeführt werden.

14. Verfahren nach Anspruch 1, wobei einer oder beide Schritte (e) und (g) unter Verwendung von Mikrofluid-Technik durchgeführt werden.

15. Verfahren nach Anspruch 1, wobei einer oder beide Schritte (e) und (g) unter Verwendung von Tintenstrahldrucker-Technologie durchgeführt werden.

16. Verfahren nach Anspruch 1, wobei die Verbindungen ausgewählt sind aus der Gruppe bestehend aus nicht-polymeren organischen Verbindungen, Lipiden, Kohlenhydraten, Peptiden, anorganischen Verbindungen und Oligonukleotiden.

17. Verfahren nach Anspruch 1, wobei mindestens eine der Verbindungen in gereinigter Form eingesetzt wird.

18. Verfahren nach Anspruch 17, wobei jede der Verbindungen in gereinigter Form eingesetzt wird.

19. Verfahren nach Anspruch 1, wobei die Verbindungen als Bestandteile von Mischungen bereitgestellt werden.

20. Verfahren nach Anspruch 19, wobei die Mischungen Exktrakte natürlicher Produkte sind.

21. Verfahren nach Anspruch 1, wobei die Wirkung eine synergistische Wirkung ist.

22. Verfahren nach Anspruch 1, wobei mindestens eine der Verbindungen ein Molekül mit einer molaren Masse von weniger als 1500 g/mol ist.

23. Verfahren nach Anspruch 22, wobei das Molekül ein Wirkstoff ist, der von der US-amerikanischen Bundesbehörde zur Überwachung von Nahrungs- und Arzneimitteln (FDA) zugelassen ist.

24. Verfahren nach Anspruch 22, wobei jede der Verbindungen ein Molekül mit einer molaren Masse von weniger als 1500 g/mol ist.

25. Verfahren nach Anspruch 24, wobei jede der Verbindungen ein Wirkstoff ist, der von der US-amerikanischen Bundesbehörde zur Überwachung von Nahrungs- und Arzneimitteln (FDA) zugelassen ist.

26. Verfahren nach Anspruch 1, wobei die Kombinationen, die auf wirksame therapeutische Kombinationen gescreent werden, Kombinationen von zwei Verbindungen sind.

27. Verfahren nach Anspruch 1, wobei die Kombinationen, die auf wirksame therapeutische Kombinationen gescreent werden, Kombinationen von drei Verbindungen sind.

28. Verfahren nach Anspruch 1, wobei die Wirkung, die auf den ganzen Zellen in dem Ganz-Zell-Test entfaltet wird, ein Transport einer Verbindung über die Zellmembran, ein elektrisches Potenzial, die Bildung eines Aktionspotenzials, eine Zellproliferation, ein Zelltod, eine Zellspezifizierung, eine Zelldifferenzierung, eine Zellmigration, eine Genexpression, Proteinspiegel, eine enzymatische Aktivität, eine Phosphorylierung, eine Methylierung, eine Acetylierung, eine Proteintranslokation in den Kern oder die Fähigkeit, eine Immunantwort hervorzurufen, ist.

29. Kombination von Verbindungen, ausgewählt aus einer paarweisen Kombination von Bepridil und Podophyllotoxin, Bepridil und Promethazin, Dipyridamol und Podophyllotoxin oder Dipyridamol und Paclitaxel.

30. Pharmazeutische Zusammensetzung enthaltend (i) eine Kombination von Verbindungen, ausgewählt aus der paarweisen Kombination von Bepridil und Podophyllotoxin, Bepridil und Promethazin, Dipyridamol und Podophyllotoxin oder Dipyridamol und Paclitaxel, und (ii) einen pharmazeutisch geeigneten Träger.

## Revendications

1. Un procédé pour le criblage de combinaisons de composés en vue d'identifier les combinaisons des composés qui provoquent un effet qui est différent de l'effet d'un composé de la combinaison seul, ce procédé comprenant les étapes de :
(a) fournir des cellules entières d'un dosage de cellules entières,
(b) contacter lesdites cellules entières avec au moins 100 composés dans des conditions qui font en sorte que chaque contact entre la cellule entière et le composé soit différent des autres,
(c) détecter ou mesurer les effets présentés par les composés sur les cellules entières,
ce procédé étant **caractérisé en ce qu'**il comprend les étapes supplémentaires suivantes :
(d) choisir des composés qui présentent une variation dudit effet produit sur les cellules entières par rapport audit effet des cellules entières non mises au contact desdits composés,
(e) création d'une matrice d'au moins 49 combinaisons uniques à deux entités ou de combinaisons d'ordre supérieur à partir des composés identifiés,
(f) mise en contact de ladite matrice de combinaisons de composés avec les cellules entières du dosage de cellules entières dans des conditions qui font que chaque contact entre la cellule entière et la combinaison du composé est différent des autres,
(g) détecter ou mesurer un effet que présentent les combinaisons des composés sur les cellules entières de l'étape (f), et
(h) identifier les combinaisons de composés qui présentent un effet de l'étape (g) différent de l'effet d'un composé de la combinaison seul.

2. Le procédé selon la revendication 1, dans lequel l'étape (e) comprend la création d'une matrice d'au moins 200 combinaisons uniques de deux ou plusieurs composés.

3. Le procédé selon la revendication 2, dans lequel l'étape (e) comprend la création d'une matrice d'au moins 400 combinaisons uniques de deux ou plusieurs composés.

4. Le procédé selon la revendication 3, dans lequel l'étape (e) comprend la création d'une matrice d'au moins 1540 combinaisons uniques de deux ou plusieurs composés.

5. Le procédé selon la revendication 1, dans lequel ladite étape de détection (g) est mise en oeuvre selon un dosage cytoblot.

6. Le procédé selon la revendication 1, dans lequel ladite étape de détection (g) est mise en oeuvre selon un dosage de gène rapporteur.

7. Le procédé selon la revendication 1, dans lequel ladite étape de détection (g) est mise en oeuvre selon un dosage de transfert d'énergie par résonance de fluorescence.

8. Le procédé selon la revendication 1, dans lequel ladite étape de détection (g) est mise en oeuvre par détection d'un colorant indicateur fluorescent de liaison calcium.

9. Le procédé selon la revendication 1, dans lequel ladite étape de détection (g) emploie une microscopie par fluorescence.

10. Le procédé selon la revendication 1, dans lequel ladite étape de détection (g) emploie le profilage d'expression.

11. Le procédé selon la revendication 1, dans lequel lesdites cellules entières sont des cellules humaines.

12. Le procédé selon la revendication 1, dans lequel lesdites cellules entières sont choisies dans le groupe comprenant les cellule du cancer, cellule immune, un neuron, un fibroblaste, les cellules bactériennes et les cellules de levure.

13. Le procédé selon la revendication 1, dans lequel une ou plusieurs des étapes (e) et (g) sont mises en oeuvre en utilisant des systèmes robotiques.

14. Le procédé selon la revendication 1, dans lequel une ou plusieurs des étapes (e) et (g) sont mises en oeuvre avec emploi de systèmes microfluidiques.

15. Le procédé selon la revendication 1, dans lequel une ou plusieurs des étapes (e) et (g) sont mises en oeuvre en utilisant une technologie d'impression à jet d'encre.

16. Le procédé selon la revendication 1, dans lequel lesdits composés sont choisis dans le groupe comprenant les composés organiques non polymères, les lipides, les hydrates de carbone, les peptides, les composés inorganiques et les oligo-nucléotides.

17. Le procédé selon la revendication 1, dans lequel au moins un desdits composés est employé sous forme purifiée.

18. Le procédé selon la revendication 17, dans lequel chacun desdits composés est employé sous forme purifiée.

19. Le procédé selon la revendication 1, dans lequel lesdits composés sont fournis sous la forme de mélanges de composés.

20. Le procédé selon la revendication 19, dans lequel lesdits mélanges sont formés d'extraits de produits naturels.

21. Le procédé selon la revendication 1, dans lequel ledit effet est un effet synergique.

22. Le procédé selon la revendication 1, dans lequel au moins un desdits composés présente un poids moléculaire inférieur à 1500 g/mole.

23. Le procédé selon la revendication 22, dans lequel ladite molécule est un médicament approuvé par la FDA.

24. Le procédé selon la revendication 22, dans lequel chacun desdits composés est une molécule présentant un poids moléculaire inférieur à 1500 g/mole.

25. Le procédé selon la revendication 24, dans lequel chacun desdits composés sont des médicaments approuvés par la FDA.

26. Le procédé selon la revendication 1, dans lequel lesdites combinaisons criblées pour former des combinaisons thérapeutiques efficaces sont des combinaisons de deux composés.

27. Le procédé selon la revendication 1, dans lequel lesdites combinaisons criblées pour former des combinaisons thérapeutiques efficaces sont des combinaisons de trois composés.

28. Le procédé selon la revendication 1, dans lequel ledit effet que présentent les cellules entières dans le dosage des cellules entières est le transport d'un composé à travers la membrane de la cellule, le potentiel électrique, la génération de potentiel d'action, la prolifération cellulaire, la mort cellulaire, la spécification cellulaire, la différenciation cellulaire, la migration cellulaire, l'expression des gènes, les taux de protéine, l'activité enzymatique, la phosphorylation, la méthylation, l'acétylation, la translocation de protéine par rapport au noyau, ou la capacité à produire une réponse immunitaire.

29. Une combinaison de composés choisie dans un groupe de combinaison formé de paires de bepridil et de podophyllotoxine, de bepridil et de prométhazine, de dipyridamole et de podophyllotoxine, ou de dipyridamole et de paclitaxel.

30. Une combinaison pharmaceutique comprenant (i) une combinaison de composés choisie dans un groupe de combinaison formé de paires de bepridil et de podophyllotoxine, de bepridil et de prométhazine, de dipyridamole et de podophyllotoxine, ou de dipyridamole et de paclitaxel, et (ii) un support pharmaceutiquement acceptable.
